# EUROPÄISCHE PATENTSCHRIFT

(11) **EP 1 940 325 B1**
(45) Veröffentlichungstag und Bekanntmachung des Hinweises auf die Patenterteilung: **21.07.2010**
(21) Anmeldenummer: 06805385.9
(22) Anmeldetag: 10.10.2006
(51) Int. Cl.: A61F 2/68, A61F 2/76

(54) **VERFAHREN ZUR DURCHFÜHRUNG EINER FUNKTIONSANALYSE AN EINER KÜNSTILICHEN EXTREMITÄT**
METHOD FOR CARRYING OUT A FUNCTIONAL ANALYSIS OF AN ARTIFICIAL EXTREMITY
PROCEDE POUR EFFECTUER UNE ANALYSE FONCTIONNELLE SUR UNE EXTREMITE ARTIFICIELLE

(30) Priorität: 26.10.2005 DE 102005051496
(43) Veröffentlichungstag der Anmeldung: 09.07.2008
(73) Patentinhaber: Otto Bock HealthCare GmbH, 37115 Duderstadt (DE)
(72) Erfinder: PUSCH, Martin, 37115 Duderstadt (DE)
(74) Vertreter: Lins, Edgar
(86) Internationale Anmeldenummer: PCT/DE2006/001767
(87) Internationale Veröffentlichungsnummer: WO 2007/048374

(56) Entgegenhaltungen:
- WO-A2-01/17466
- WO-A2-2004/041132
- DE-A1- 10 139 333
- DE-A1- 19 754 690

## Beschreibung

Die Erfindung betrifft ein Verfahren zur Durchführung einer Funktionsanalyse an einer mit einer künstlichen Extremität ausgerüsteten Person, wobei die künstliche Extremität in in einer Grundfunktion einstellbar und modular mit wenigstens einem ausbaubaren Funktionsteil zur Ermöglichung einer Zusatzfunktion ausgebildet ist. Ein solches Verfahren ist z.B. aus dem Dokument DE-A-19754 690 bekannt.

Künstliche Extremitäten sind in sehr hoch entwickelten Ausführungsformen im Einsatz. Für die unter Sicherheitsaspekten des Patienten besonders relevanten Prothesen für untere Extremitäten unter Einschluss eines künstlichen Kniegelenks werden beispielsweise für die Funktionen "Gehen" und "Stehen" ausgefeilte Technologien eingesetzt, die einerseits ein sicheres Stehen und andererseits einen dem natürlichen Gehen möglichst gut angepassten Bewegungsablauf beim Gehen ermöglichen. Hierzu ist es beispielsweise erforderlich, unter Berücksichtigung der üblichen Auslösekraft des Patienten, beispielsweise über die Bewegung des Oberschenkelstumpfes, ein vollständiges Vorholen des Unterschenkels zu ermöglichen und dabei jedoch ein heftiges Anschlagen des Unterschenkelteils an einen die Streckbewegung begrenzenden Anschlag zu vermeiden. Ersichtlich werden dabei ggf. progressive Dämpfungen eingesetzt, die die gewünschte Funktion jedoch nur gewährleisten, wenn sie in ihrer Dämpfungsdosierung für den jeweiligen Patienten richtig eingestellt sind. Ähnliches gilt für die Auslösung der Gehbewegung aus dem Stehen heraus und für den Übergang vom Gehen zum sicheren Stehen.

Es ist bereits bekannt, die Funktion einer derartigen Prothese durch Sensoren zu steuern, die den Übergang von einer Phase der Gehbewegung in eine andere Phase oder den Übergang von der Gehbewegung in eine Stehbewegung und umgekehrt aufgrund von gemessenen Kräften, Beschleunigungen, Momenten o. dgl. ermitteln und Einstellungen der Prothese für die nächste Funktionsphase durchführen. Ein Beispiel für eine derartige Prothese ist die unter der Bezeichnung C-Leg der Anmelderin entwickelte und vertriebene Beinprothese. Auch eine derartige, hoch entwickelte Prothese benötigt jedoch Einstellungsvorgänge, um die Anpassung der Funktion der Prothese an den jeweiligen Patienten zu optimieren. Derartige Einstellungen können unter Berücksichtigung der subjektiven Eindrücke des Patienten bei der Benutzung der Prothese vorgenommen werden. Nachteilig hieran ist jedoch, dass die subjektiven Eindrücke des Patienten schwanken und dass eine Quantifizierung der Eindrücke kaum möglich ist. Die Optimierung der Einstellung der Prothese muss daher nach dem Versuch- und Irrtum-Prinzip erfolgen, um sich so einer optimierten Einstellung anzunähern.

Es sind Vorrichtungen bekannt, die eine Stand- und Ganganalyse des mit der Prothese versorgten Patienten in objektivierter Form ermöglichen. Hierfür sind aufwändige und daher teure Messsysteme erforderlich, die nur in wenigen Labors, beispielsweise in Rehabilitationszentren, vorgehalten werden können. Für die Standardanpassung einer Prothese durch den Orthopädiemechaniker sind derartige Messsysteme unerschwinglich, sodass die Standardanpassung ohne Zuhilfenahme derartiger Messsysteme, also im Wesentlichen auf der Basis der subjektiven Eindrücke des Patienten, erfolgt.

Der vorliegenden Erfindung liegt die Aufgabe zugrunde, eine Funktionsanalyse einer künstlichen Extremität ohne aufwändige Messapparaturen zu ermöglichen.

Zur Lösung dieser Aufgabe ist erfindungsgemäß ein Verfahren der eingangs erwähnten Art dadurch gekennzeichnet, dass anstelle des ausbaubaren Funktionsteils eine an die für den Einbau des Funktionsteils relevanten Abmessungen angepasste Sensoranordnung eingebaut wird, dass mit der Sensoranordnung Kräfte, Beschleunigungen und/oder Momente während der Benutzung der künstlichen Extremität gemessen und zur Optimierung der Einstellung der Grundfunktion der künstlichen Extremität verwendet werden und dass die Sensoranordnung durch das Funktionsteil unter Beibehaltung der optimierten Einstellung ersetzt wird.

Die vorliegende Erfindung ermöglicht somit eine Durchführung von Messungen an der vollständig funktionsfähigen Prothese, die für die Benutzung durch den Patienten vorgesehen und an den Patienten angepasst ist. Die Prothese kann daher über eine beliebig lange Zeit von dem Patienten mit der eingebauten Sensoranordnung benutzt werden, um durch Auswertung der Messwerte feststellen zu können, ob die Einstellung der Prothese noch optimierbar ist und ob beispielsweise bei einer längeren Benutzung der Prothese Änderungen des Gangverhaltens des Patienten eintreten und daher andere Einstellungen der Prothese zweckmäßig wären.

Hierin liegt der wesentliche Unterschied zu speziellen Messprothesen, die üblicherweise verwendet werden, um erste Messwerte für einen Patienten zur Erstellung einer angemessenen Prothese zu erhalten. Eine derartige Messprothese ist nicht an den Patienten individuell angepasst und kann daher nur zum Erhalt erster Anhaltspunkte für den speziellen Patienten dienen. Demgegenüber wird erfindungsgemäß die für den Patienten erstellte und vollständig angepasste Prothese in voller Funktionsfähigkeit bezüglich der Grundfunktion der Prothese verwendet.

Durch eine kompakt aufgebaute und zuverlässige Daten ermittelnde Sensoranordnung ist es im Rahmen der Erfindung möglich, ein für eine Zusatzfunktion vorgesehenes Funktionsteil der Prothese auszubauen und durch die in ihren Einbauabmessungen angepasste Sensoranordnung zu ersetzen. Selbstverständlich muss die Sensoranordnung dabei so ausgebildet sein, dass sie die Messungen ohne für den Gebrauch merkliche Relativbewegungen auskommt. Bevorzugt ist daher die Ausbildung der Sensoranordnung mit Dehnungsmessstreifen, Piezoelementen o. ä., bei denen Relativbewegungen bzw. Verformungen im Bereich von Bruchteilen eines Millimeters für die Generierung der Messsignale ausreichen.

Ein Beispiel für ein ausbaubares Funktionsteil an einer Beinprothese ist ein Drehadapter, der unmittelbar oberhalb des Kniegelenks eingesetzt wird, um insbesondere im Sitzen eine Drehung des Unterschenkels relative zum Oberschenkel zu ermöglichen, wodurch beispielsweise eine Sitzhaltung mit überschlagenden Beinen erleichtert wird. Für die Durchführung der Funktionsanalyse kann der Patient auf diese Zusatzfunktion, die die Grundfunktion der Beinprothese, nämlich während des Gehvorganges und während des Stehzustandes, nicht beeinflusst, bequem verzichten. Selbstverständlich ist es aber auch möglich, bei einer modular aufgebauten Prothese ein das Unterschenkelteil und das Oberschenkelteil bildende Rohrstück um genau das Maß zu verkürzen, das für den Einbau der Sensoranordnung benötigt wird. Bei einem Ausbau der Sensoranordnung kann dann an das verkürzte Rohrstück ein entsprechendes Verlängerungsstück arigeflanscht oder das verkürzte Rohrstück durch ein längeres Rohrstück ersetzt werden,

Das durch die Sensoranordnung ersetzte Funktionsteil kann auch ein tragendes Modulteil, wie z. B. ein Rohrmodul sein. Das ersetzte Modulteil ist nicht bestimmend für die Grundfunktion, wie dies beispielsweise ein Kniegelenk oder die den Abrollvorgang bestimmenden Teil eines künstlichen Fußes wären. Die Sensoranordnung kann somit auch in Kombination mit einem Rest-Modulteil ausgebildet sein, wobei Gewicht und Gewichtsverteilung dem ersetzten Modulteil im Wesentlichen entsprechen sollten. Ein Beispiel für eine derartige Sensoranordnung ist ein Messsensor mit einem Rest-Unterschenkelrohr, wodurch ein komplettes Unterschenkelrohr einer Beinprothese ersetzt wird.

Obwohl in der obigen Beschreibung in erster Linie auf eine Prothese für eine untere Extremität eingegangen worden ist, ist es ohne weiteres ersichtlich, dass die Erfindung auch für den Einsatz von Prothesen für die oberen Extremitäten, also Armprothesen, Handprothesen und Schulterprothesen mit Vorteil einsetzbar ist.

In allen Fällen können durch die Sensoranordnung selbst oder auch durch zusätzlich in die Prothese eingesetzte Sensoren zusätzliche Messdaten gewonnen werden, wie beispielsweise Drehraten, Winkellagen und Winkeländerungen usw.

Die Erfindung soll im Folgenden anhand eines in der Zeichnung dargestellten Ausführungsbeispiels und der Beschreibung einer für die Durchführung der Erfindung besonders geeigneten Sensoranordnung näher erläutert werden. Es zeigen:
- Figur 1: eine perspektivische Seitenansicht auf ein erstes Teil einer Sensoranordnung;
- Figur 2: eine perspektivische Ansicht von schräg unten auf das erste Teil gemäß Figur 1;
- Figur 3: eine perspektivische Seitenansicht des zweiten Teils einer Sensoranordnung;
- Figur 4: eine perspektivische Ansicht von schräg unten des zweiten Teils gemäß Figur 3;
- Figur 5: eine perspektivische Seitenansicht der aus den beiden Tei- len zusammengesetzten Sensoranordnung;
- Figur 6: eine perspektivische Ansicht von schräg unten der Sensor- anordnung gemäß Figur 5;
- Figur 7: eine schematische Schnittdarstellung der Sensoranordnung für eine axiale Belastung (z-Richtung) mit einer schemati- schen Darstellung linienförmiger Stauchbereiche auf dem zweiten Flansch;
- Figur 8: eine Darstellung gemäß Figur 7 für ein Drehmoment um eine horizontale Achse (x-Achse);
- Figur 9: eine schematische Darstellung gemäß Figur 7 für eine ein- wirkende Seitenkraft;
- Figur 10: eine schematische Darstellung gemäß Figur 7 für ein Dreh- moment um eine vertikale Achse (z-Achse);
- Figur 11: eine Seitenansicht einer Beinprothese mit einem Drehadap- ter;
- Figur 12: eine Seitenansicht gemäß Figur 11, in der der Drehadapter durch die Sensoranordnung gemäß den Figuren 1 bis 10 er- setzt worden ist.

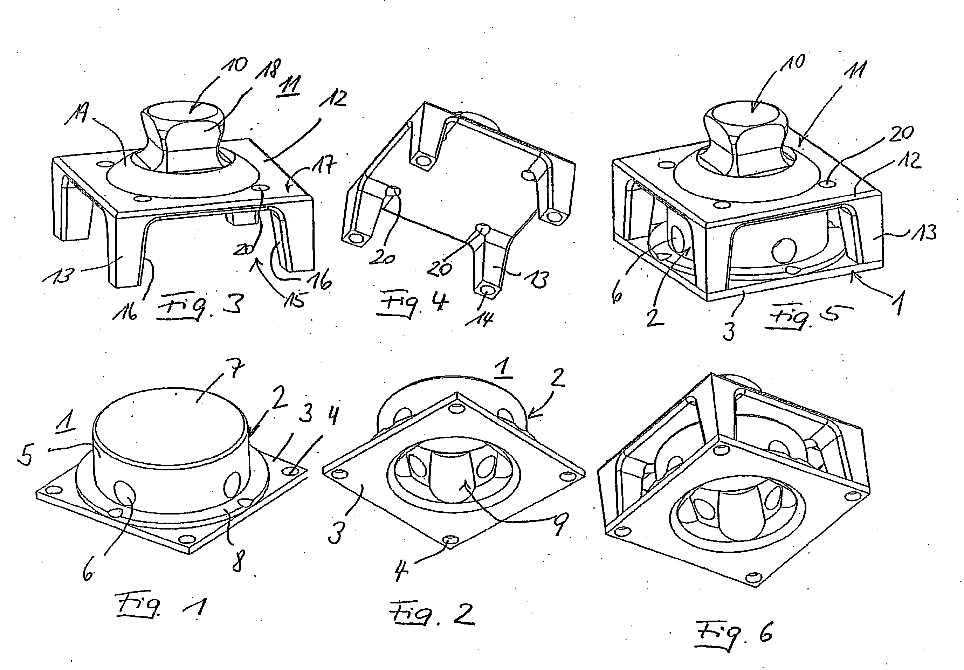

Die Figuren 1 bis 6 verdeutlichen den Aufbau eines Ausführungsbeispiels einer erfindungsgemäßen Sensoranordnung. Diese besteht aus einem erste Teil 1, das aus einem hutförmigen zylindrischen Anschluss 2 und einem sich daran anschließenden quadratischen Flansch 3 aufgebaut ist. Der quadratische Flansch 3 weist an seinen Ecken Durchgangslöcher 4 für (nicht dargestellte) Befestigungsschrauben auf.

Der hutförmige Anschluss 2 ist mit einer zylindrischen Mantelwandung 5 aufgebaut, in der sich, jeweils um einen Drehwinkel von 90° versetzt, Gewindebohrungen 6 befinden. Der hutförmige Anschluss 2 weist an seiner Oberseite einen kreiszylindrischen Boden 7 und an seiner Unterseite eine kreisringförmige Krempe 8 auf, die einstückig mit dem rechteckigen Flansch 3 verbunden ist und diesen verstärkt.

Figur 2 verdeutlicht, dass der hutförmige Anschluss 2 einen angenähert rechteckig ausgebildeten Aufnahmeraum 9 aufweist, der zur Aufnahme eines Justieradapters dient der vier schräg liegende Justierflächen aufweist, gegen die die durch die Gewindebohrungen 6 hindurch geschraubten Justierschrauben drücken.

Ein derartiger Justieradapter 10 ist an einem zweiten Teil 11 der Sensoranordnung ausgebildet. Das zweite Teil 11 weist einen zweiten, quadratischen Flansch 12 auf, dessen Abmessungen den Abmessungen des ersten Flansches 3 entsprechen. Die beiden Flansche 3, 12 werden durch am zweiten Teil einstückig angeformte Stege 13 miteinander verbunden, die sich an den Ecken des zweiten Flansches 12 nach unten erstrecken, sodass die Stege radial außen von dem hutförmigen Anschluss 2 auf dem ersten Flansch 3 aufliegen. Die Stege 13 sind jeweils mit einer Gewinde-Sackbohrung von ihrer Unterseite aus versehen, die mit den Durchgangsbohrungen 4 des ersten Flansches 3 fluchten können.

Die Figuren 3 und 4 lassen noch erkennen, dass die Stege 12 einen rechteckigen Querschnitt aufweisen und sich zu ihren freien Enden hin, also nach unten durch eine zu einem Zwischenraum 15 zwischen zwei Stegen 13 zeigende Schräge 16 verjüngen.

Der Justieradapter 10 befindet sich auf der von den Stegen 13 abgewandten Oberseite 17 des zweiten Flansches 12. Er ist in an sich bekannter Weise in Form eines umgekehrten Pyramidenstumpfes ausgebildet und weist somit vier schräg verlaufende plane Justierflächen 18 auf, die zum Zwecke der Justierung mit Justierschrauben zusammenwirken können. Der Justieradapter 10 geht in einen Sockel 19 mit vergrößertem Durchmesser über, der mit einer gewölbten Fläche einen Übergang zu dem quadratischen zweiten Flansch 12 herstellt.

Der Justieradapter 10 bildet einen zweiten Anschluss der Sensoranordnung. Zwischen diesem zweite Anschluss 10 und den in den Ecken des zweiten Flansches 12 angeordneten Stegen 13 befindet sich, somit in Diagonalrichtung des zweiten quadratischen Flansches 12, jeweils eine Ausnehmung 20 in Form einer Durchgangsbohrung, durch die die unten näher beschriebene Ausbildung von Spannungs- bzw. Dehnungsbereichen beeinflussbar ist.

Die Figuren 5 und 6 zeigen die aus den beiden Teilen 1, 11 zusammengesetzte Sensoranordnung im montierten Zustand (jedoch ohne Befestigungsschrauben). Es ist erkennbar, dass zwischen dem Aufnahmeraum 9 des ersten Anschlusses zwei und dem den zweiten Anschluss bildenden Justieradapter 10 nur eine geringe Bauhöhe von 2 bis 3 cm benötigt werden.

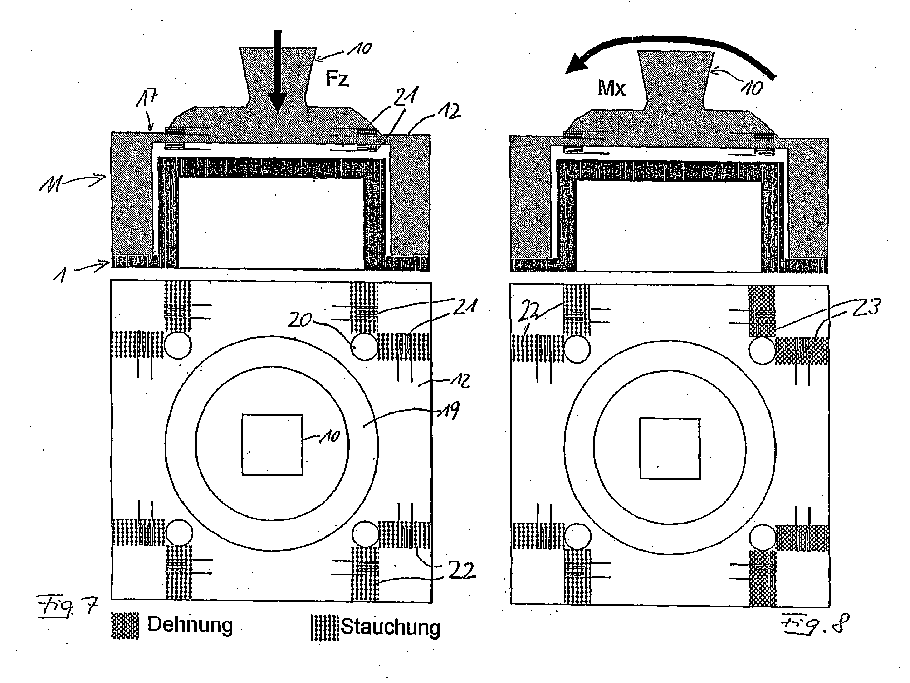

Die Figuren 7 bis 10 zeigen schematisch jeweils einen Vertikalschnitt durch die Sensoranordnung gemäß den Figuren 1 bis 6, jedoch mit einer schematischen Darstellung der auf beide Oberflächen des Flansches 12 geklebten Dehnungsmessstreifen 21 als Sensorelemente.

Die in den Figuren 7 bis 10 jeweils darunter befindliche Draufsicht verdeutlicht die Positionierung der Dehnungsmessstreifen 21 derart, dass sie durch linienförmige Stauchungsbereiche 22 oder Dehnungsbereiche 23 in ihrer Länge verändert werden, sodass sich ein geänderter Widerstand ergibt.

Figur 7 verdeutlicht den Fall einer Krafteinwirkung in z-Richtung, also in Axialrichtung einer Rohrskelettprothese für einen Unterschenkel. Die an der Oberseite 17 des zweiten Flansches 12 befindlichen Dehnungsmessstreifen 21 befinden sich dabei in Stauchungsbereichen 22, die sich linienförmig von den Durchgangsbohrungen 20 parallel zu den Kanten des zweiten Flansches 12 jeweils zur benachbarten Kante hin erstrecken. Die dementsprechend ausgerichteten Dehnungsmessstreifen 21 verändern somit ihren Widerstandswert in Richtung Stauchung.

Gemäß Figur 8 wird der Justieradapter 10 mit einem Drehmoment um eine senkrecht zur Zeichenebene (x-Richtung) stehende Achse beaufschlagt. Das Drehmoment führt für die auf der Oberseite 17 befindlichen Dehnungsmessstreifen 21 auf der Seite, zu der das Drehmoment hin gerichtet ist (vgl. den eingezeichneten Pfeil Mx in Figur 8) zu einer Stauchung, während sie auf der gegenüberliegenden Seite zur Ausbildung von Dehnungsbereichen 23 führt.

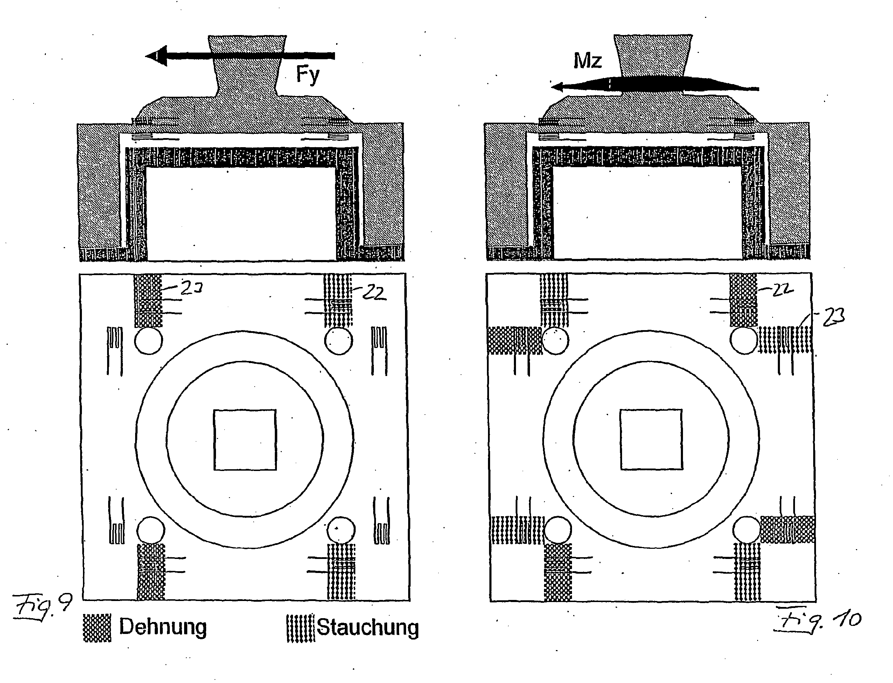

Figur 9 zeigt eine auf den Justieradapter 10 einwirkende seitliche Kraft in der Zeichenebene (y-Richtung), durch die nur senkrecht zu der angreifenden Kraft stehende Dehnungsbereiche 23 und Stauchungsbereiche 22 gebildet werden, während die übrigen Dehnungsmessstreifen 21 auf der Oberseite 17 des zweiten Flansches 12 ohne Messsignal bleiben.

Bei einem in Figur 10 dargestellten angreifenden Drehmoment Mz in z-Richtung entstehen an jeder Ausnehmung 20 ein Stauchungsbereich 23 und ein Dehnungsbereich 22, wobei in Richtung des Drehmoments Mz gesehen, der Stauchungsbereich 23 jeweils um 90° dem Dehnungsbereich 22 vorläuft.

Anhand der dargestellten Beispiele ist erkennbar, dass mit den Dehnungsmessstreifen 21 als Sensorelemente die verschiedenen auftretenden Kräfte und Momente eindeutig detektierbar sind.

Die Dehnungsmessstreifen 21 an der Unterseite des zweiten Flansches 12 ergeben jeweils Signale, die komplementär zu den Signalen der Dehnungsmessstreifen 21 an der Oberseite 17 des zweiten Flansches 12 sind, sodass sie bei einer geeigneten Addition zu einem verstärkten Messsignal beitragen können:

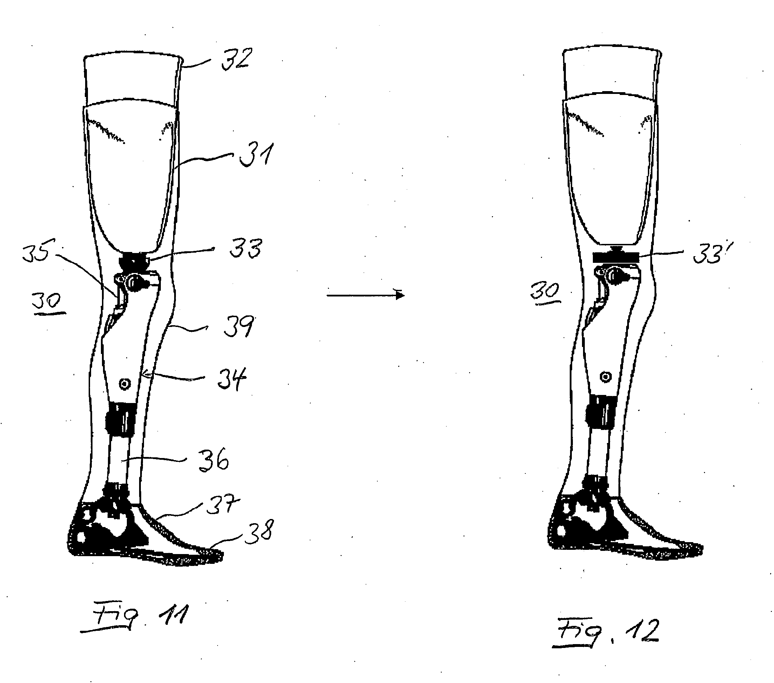

Figur 11 zeigt eine bekannte Beinprothese 30 mit einem Aufnahmetrichter 31 für einen Oberschenkelstumpf. In den Aufnahmetrichter ist ein hautfreundlicher Liner 32 eingesetzt, der einen beschwerdefreien Kontakt zum Oberschenkelstumpf herstellt.

An das untere Ende des Aufnahmetrichters 31 ist ein Drehadapter über eine übliche Justierpyramide angeschlossen. Die Verbindung des Drehadapters 33 mit einem Unterschenkelteil 34 erfolgt ebenfalls über eine Justierpyramide.

Das Unterschenkelteil 34 ist als computergesteuertes Prothesenteil ausgebildet, wie es unter der Bezeichnung "C-Leg" der Otto Bock HealthCare GmbH bekannt ist. Das Unterschenkelteil 34 enthält ein polyzentrisches Kniegelenk 35, das als Viergelenkkette in bekannter Form ausgebildet ist. An das Unterschenkelteil 34 schließt sich nach unten ein Modulrohr 36 an, das eine Verbindung zu einem künstlichen gelenklosen Fuß 37 herstellt, dessen möglicher Aufbau ebenfalls bekannt ist und hier nicht näher erläutert werden muss. Sowohl der künstliche Fuß 37 als auch die Beinprothese sind mit einem kosmetischen Überzug 38, 39 versehen.

Die Hauptfunktion der Prothese 30 besteht in der Ermöglichung eines möglichst natürlichen Gangverhaltens und eines sicheren Stehens sowie eines bequemen Hinsetzens für den individuellen Nutzer der Prothese 30. Der Drehadapter 33 ist während der Hauptfunktion der Prothese 30 verriegelt und kann entriegelt werden, wenn auf das Unterschenkelteil 34 keine Belastung ausgeübt wird. Durch den Drehadapter 33 kann der Unterschenkel der Prothese 30 gegenüber dem Oberschenkel verdreht werden, insbesondere wenn der Nutzer der Prothese 30 sitzt.

Für das Anpassen der Prothese 30 und ggf. in einem späteren Stadium für die Überprüfung der Prothese 30 kann der Drehadapter 33 durch die hier mit 33' bezeichnete Sensoranordnung ersetzt werden, wie sie in den Figuren 1 bis 10 erläutert worden ist. Durch den Austausch des Drehadapters 33 gegen die Sensoranordnung 33' bleibt die Grundfunktion der Prothese 30 unverändert, wenn die Einbaumaße der Sensoranordnung 33' denjenigen des Drehadapters 33 entsprechen. Es entfällt lediglich die Zusatzfunktion der Verdrehung des Unterschenkels gegenüber dem Oberschenkel, wodurch jedoch die Hauptfunktion der Prothese 30, nämlich das Verhalten beim Gehen, Stehen und Hinsetzen nicht beeinträchtigt wird. Mittels der Sensoranordnung 33' können daher die für eine Auswertung der Funktion der Prothese 33 benötigten Daten an der für den Patienten individuell angepassten und voll funktionstüchtigen Prothese 30 ermittelt werden. Die Sensoranordnung 33' ist dabei für die erste Anpassung der Prothese, also für einen Kurzzeiteinsatz, ebenso brauchbar wie für eine Langzeituntersuchung der Bewegung des Patienten mit der speziell für ihn angepassten und eingestellten Prothese 30.

## Patentansprüche

1. Verfahren zur Durchführung einer Funktionsanalyse an einer mit einer künstlichen Extremität (30) ausgerüsteten Person, wobei die künstliche Extremität (30) in einer Grundfunktion einstellbar und modular mit wenigstens einem ausbaubaren Funktionsteil (33), insbesondere zur Ermöglichung einer Zusatzfunktion, ausgebildet ist, **dadurch gekennzeichnet, dass** anstelle des ausbaubaren Funktionsteils (33) eine an die für den Einbau des Funktionsteils relevanten Abmessungen angepasste Sensoranordnung (33') eingebaut wird, dass mit der Sensoranordnung (33') Kräfte, Beschleunigungen und/oder Momente während der Benutzung der künstlichen Extremität (30) gemessen und zur Optimierung der Einstellung der Grundfunktion der künstlichen Extremität verwendet werden und dass die Sensoranordnung (33') durch das Funktionsteil (33) unter Beibehaltung der optimierten Einstellung ersetzt wird.

2. Verfahren nach Anspruch 1, **dadurch gekennzeichnet, dass** als künstliche Extremität (30) eine Beinprothese verwendet wird.

3. Verfahren nach Anspruch 2, **dadurch gekennzeichnet, dass** die Sensoranordnung (33') an die Abmessungen eines oberhalb eines künstlichen Kniegelenks angeordneten Drehadapters (33) angepasst wird.

## Claims

1. A method for carrying out a functional analysis on a person equipped with an artificial extremity (30), the artificial extremity (30) in a basic function being adjustable, and said artificial extremity (30) being formed in a modular fashion with at least one removable functional part (33), in particular to permit an additional function, **characterized in that** a sensor assembly (33'), which is adapted to the dimensions relevant for the installation of the functional part, is installed in place of the removable functional part (33), **in that** forces, accelerations and/or torques are measured by means of the sensor assembly (33') during the use of the artificial extremity (30) and are used to optimize the setting of the basic function of the artificial extremity, and **in that** the sensor assembly (33') is replaced by the functional part (33) while retaining the optimized setting.

2. The method as claimed in claim 1, **characterized in that** a leg prosthesis is used as artificial extremity (30).

3. The method as claimed in claim 2, **characterized in that** the sensor assembly (33') is adapted to the dimensions of a rotational adaptor (33) arranged above an artificial knee joint.

## Revendications

1. Procédé pour effectuer une analyse fonctionnelle sur une personne équipée d'une extrémité (30) artificielle, l'extrémité (30) artificielle étant réglable dans une fonction fondamentale et étant formée modulairement avec au moins une partie fonctionnelle (33) démontable, en particulier pour permettre une fonction additionnelle, **caractérisé en ce qu'**à la place de la partie fonctionnelle (33) démontable, on monte un dispositif de détection (33') adapté aux dimensions pertinentes pour le montage de la partie fonctionnelle, **en ce qu'**à l'aide du dispositif de détection (33'), pendant l'utilisation de l'extrémité (30) artificielle, on mesure des forces, accélérations et/ou moments et on les utilise pour optimiser le réglage de la fonction fondamentale de l'extrémité artificielle, et **en ce que** l'on remplace le dispositif de détection (33') par la partie fonctionnelle (33) en maintenant le réglage optimisé.

2. Procédé selon la revendication 1, **caractérisé en ce que** l'on utilise en tant qu'extrémité (30) artificielle une prothèse de jambe.

3. Procédé selon la revendication 2, **caractérisé en ce que** l'on adapte le dispositif de détection (33') aux dimensions d'un adaptateur de rotation (33) agencé au-dessus d'une articulation de genou artificielle.
